# EUROPÄISCHE PATENTSCHRIFT

(11) **EP 1 056 713 B1**
(45) Veröffentlichungstag und Bekanntmachung des Hinweises auf die Patenterteilung: **25.06.2003**
(21) Anmeldenummer: 99908908.9
(22) Anmeldetag: 16.02.1999
(51) Int. Cl.: C07C 213/00

(54) **VERFAHREN ZUR HERSTELLUNG WÄSSRIGER, IM WESENTLICHEN METALLIONENFREIER HYDROXYLAMINLÖSUNGEN**
METHOD FOR PRODUCING AQUEOUS HYDROXYLAMINE SOLUTIONS WHICH ARE SUBSTANTIALLY FREE OF METAL IONS
PROCEDE POUR PRODUIRE DES SOLUTIONS AQUEUSES D'HYDROXYLAMINE, PRATIQUEMENT EXEMPTES D'ION METALLIQUE

(30) Priorität: 17.02.1998 DE 19806578
(43) Veröffentlichungstag der Anmeldung: 06.12.2000
(73) Patentinhaber: BASF AKTIENGESELLSCHAFT, 67056 Ludwigshafen (DE)
(72) Erfinder: WATZENBERGER, Otto, D-68199 Mannheim (DE); SCHNEIDER, Hans-Michael, D-67549 Worms (DE)
(74) Vertreter: Kinzebach, Werner, Dr.
(86) Internationale Anmeldenummer: EP9900993
(87) Internationale Veröffentlichungsnummer: WO99042434

(56) Entgegenhaltungen:
- EP-A- 1 017 621
- WO-A-97/22551
- WO-A-99/03780
- DE-A- 3 608 495
- US-A- 4 147 623
- US-A- 5 472 679
- US-A- 5 788 946
- CHEMICAL ABSTRACTS, vol. 81, no. 26, 30. Dezember 1974 (1974-12-30) Columbus, Ohio, US; abstract no. 172427h, HOROSE TOSHIYOSHI: "Stabilizing an aqueous solution of hydroxylamine or a salt thereof" Seite 177; Spalte 2; XP002900526 & JP 49 014640 B (TOA GOSEI CHEM. IND. LTD.) 9. April 1974 (1974-04-09)

## Beschreibung

Die vorliegende Erfindung betrifft ein Verfahren zur Herstellung wäßriger Hydroxylaminlösungen, die im wesentlichen metallionenfrei sind.

Hochreine, konzentrierte, wäßrige Hydroxylaminlösungen werden unter anderem in der Elektronikindustrie, beispielsweise zusammen mit anderen Stoffen zum Vorreinigen der Platinen, verwendet. Für die Verwendung in der Elektronikindustrie wird üblicherweise ein Gehalt an Verunreinigungen weit unter 1 ppm, im allgemeinen sogar im ppb-Bereich, gefordert (sogenannte "Electronic grade"- Ware). Die derzeit im Handel erhältlichen Hydroxylaminlösungen enthalten jedoch herstellungsbedingt Verunreinigungen im ppm-Bereich, wie beispielsweise Natriumsulfat oder andere Metallverbindungen.

Eine Möglichkeit der Reinigung besteht in der destillativen Aufarbeitung, wie sie in der US-A-5,472,679 beschrieben ist. Allerdings muß bei der Destillation darauf geachtet werden, daß die Temperatur von 65°C nicht überschritten wird, weil die Onset-Temperatur, d.h. diejenige Temperatur, bei der eine erkennbare Zersetzung beginnt, bei einer 50 gew.-%igen Hydroxylaminlösung bei ca. 70°C liegt. Um Hydroxylamin als Kopfprodukt abtrennen zu können, destilliert man üblicherweise in kleinem Maßstab und im Vakuum bei sehr niedrigen Temperaturen. Eine derartige Destillation ist mit sehr hohem Aufwand und Zeitbedarf verbunden.

Die in der WO 97/22551 beschriebene destillative Aufarbeitung vermeidet die Nachteile des in der genannten US-Patentschrift beschriebenen Verfahrens. Dennoch gilt auch für das Verfahren der WO 97/22551, daß ein relativ hoher Aufwand erforderlich ist. Dementsprechend sind salzfreie wäßrige Hydroxylaminlösungen in Electronic grade-Reinheit entsprechend teuer, so daß ihre Anwendung wirtschaftlich auf wenige Einsatzbereiche beschränkt ist.

Der vorliegenden Erfindung liegt daher die Aufgabe zugrunde, ein Verfahren zur Herstellung von Hydroxylaminlösungen zur Verfügung zu stellen, die im wesentlichen frei an Metallionen, insbesondere Natriumionen, sind, wobei das Verfahren einfach und in wirtschaftlicher Weise durchführbar sein soll.

Die Anwendung von Kationenaustauschern zum Abtrennen von Metallionen aus wäßrigen Lösungen ist bekannt. Typische Anwendungsbereiche liegen im Bereich der Wasserreinigung, wobei Metallionen in der Regel unselektiv abgetrennt werden. Ziel ist üblicherweise die Herstellung einer im wesentlichen metallionenfreien Lösung. Weiter sind Kationenaustauscher bekannt, die mehrwertige Metallionen, welche leicht Komplexe bilden, wie beispielsweise Fe³⁺ oder Ni²⁺, selektiv aus wäßrigen Lösungen abzutrennen vermögen. Dies beruht darauf, daß diese Metallionen Komplexe mit dem Ionenaustauscher bilden, der chelatbildende Gruppen aufweist, wie beispielsweise in "The Many Faces of Ion-Exchange Resins, Chemical Engineering, June 1997, 94-100, berichtet wird. Darin ist in der Tabelle auf Seite 98 angegeben, daß Fe³⁺-Ionen eine 350000-fach höhere, Ni-Ionen eine 3200-fach höhere Affinität zu dem beschriebenen Ionenaustauscher besitzen als die Vergleichssubstanz Ca²⁺. Bei Anwesenheit von etwa 50 g/l Ammoniumionen (entsprechend 200 g/l Ammoniumsulfat) fallen die Affinitätswerte stark ab, die Affinität von Nickelionen beispielsweise auf 30. Alkalimetallkationen sind in dieser Tabelle nicht enthalten. Sie besitzen eine noch viel geringere Affinität als die in der Tabelle zum Vergleich verwendeten Calciumionen.

Wäßrige Hydroxylaminlösungen enthalten neben den als Verunreinigung vorhandenen Metall auch das durch Aufnahme eines Protons gebildete Hydroxylaimnoniumkation. Bei einer 50 gew.-%igen Hydroxylaminlösung sind 15,14 Mol Hydroxylamin/l neben 1 bis 10 ppm Metallionen (entsprechend 0,4 bis 1,7 x 10⁻⁵ Mol/l, bezogen auf Na⁺-Ionen), vorhanden. Die abzutrennenden Kationen liegen gegenüber den Hydroxylammoniumkationen im Unterschuß vor. Unter diesen Umständen erwartet der Fachmann, daß eine weitere Abreicherung der in diesen geringen Mengen enthaltenen Metallionen und insbesondere Alkalimetallionen, durch Behandlung mit einem Ionenaustauscher nicht mehr möglich ist.

Überraschenderweise wurde aber nun gefunden, daß durch Behandlung der Hydroxylaminlösungen mit einem sauren Kationenaustauscher die Metallionen selektiv abgetrennt werden können und die oben genannte Aufgabe gelöst wird.

Die WO 99/03780 (die nach dem für die vorliegende Anmeldung beanspruchten Prioritätstag veröffentlicht wurde) offenbart ein Verfahren zur Reinigung von Hydroxylamin. Eine Hydroxylaminlösung wird über ein Bett eines stark sauren Ionenaustauscherharzes und dann über ein Bett eines stark basischen Anionenaustauscherharzes geleitet. Der stark saure Ionenaustauscher wird mit verdünnter Salzsäure vorbehandelt.

Chemical Abstracts, Vol. 81, No. 26, Abstract No. 172427h beschreibt die Stabilisierung einer wässrigen Lösung von Hydroxylamin oder eines Salzes davon, indem der pH auf 2 bis 7 eingestellt wird und die Lösung mit einem Kationenaustauscherharz behandelt wird.

Die US 4,147,623 beschreibt ein Verfahren zur Abtrennung von Hydroxylamin aus wässrigen Lösungen, die neben Hydroxylammoniumionen Salze von Kationen enthalten, deren korrespondierende Basen Dissoziationskonstanten von wenigstens 10⁻⁷ aufweisen. Die Lösung wird auf einen pH von 6 bis 11 gebracht und über ein Bett eines Kationenaustauscherharzes geleitet, das mit ladungskompensierenden Kationen beladen ist. Die ionischen Salze werden zuerst eluiert.

In einem ersten Aspekt betrifft die Erfindung ein Verfahren zur Herstellung einer wäßrigen Hydroxylaminlösung, die im wesentlichen metallionenfrei ist, dadurch gekennzeichnet, daß man die Hydroxylaminlösung mindestens einer Behandlung mit einem Anionenaustauscher in Hydroxyl-Form unterwirft, die behandelte Hydroxylaminlösung mit einem Stabilisator versetzt und die Hydroxylaminlösung anschließend mindestens einer Behandlung mit einem sauren Kationenaustauscher unterwirft.

In einem zweiten Aspekt betrifft die Erfindung ein Verfahren zur Herstellung einer wäßrigen Hydroxylaminlösung, die im wesentlichen metallionenfrei ist, dadurch gekennzeichnet, daß man die Hydroxylaminlösung mindestens einer Behandlung mit einem Anionenaustauscher in Hydroxyl-Form unterwirft, die Hydroxylaminlösung anschließend mindestens einer Behandlung mit einem sauren Kationenaustauscher unterwirft und die Hydroxylaminlösung mit einer wäßrigen Lösung eines Stabilisators versetzt, die zuvor zur Entfernung von Metallionen einer Behandlung mit einem Kationenaustauscher unterworfen wurde.

Vorzugsweise verwendet man zu diesem Zweck einen schwach sauren Kationenaustauscher, d.h. einen Kationenaustauscher mit einem pH in der säureform im Bereich von 2 bis 6, insbesondere 3 bis 6.

Weiterhin bevorzugt verwendet man einen Kationenaustauscher, der chelatbildende Gruppen, wie Iminodiessigsäuregruppen, aufweist.

Brauchbare Kationenaustauscher sind beispielsweise die Lewatit TP-Typen der Firma Bayer, wie Lewatit TP 207, die Amberlite IRC-Typen, Duolite C 433 etc., Dowex CCR oder MWC u. dgl. Die Kationenaustauscher kommen in der Säureform zur Anwendung. Gegebenenfalls werden sie zu diesem Zweck mit einer Säure, beispielsweise Schwefelsäure behandelt, um das Kation zu entfernen. Danach wird üblicherweise mit hochreinem Wasser, säurefrei gespült.

Die Behandlung der Hydroxylaminlösung kann auch mit einem stark sauren Kationenaustauscher in der Säureform erfolgen, d. h. einem Kationenaustauscher mit einem pH in der Säureform im Bereich von 0 bis 2, insbesondere 0 bis 1. Brauchbare stark saure Kationenaustauscher sind beispielsweise die Harze Amberlite IR-120, IR-122, IRC-50 und Amberjet 1500H der Fa. Rohm & Haas, Dowex 88 von Dow Chemical, Duolite C-200, C-26 und C-280 der Fa. Rohm und Haas sowie Purolite C-100, C-105 und C-150. Die Säureform kann mit üblichen starken Säuren, wie Salzsäure, erzeugt werden.

Die Behandlung der Hydroxylaminlösung mit dem Kationenaustauscher erfolgt in üblicher Weise, beispielsweise durch Behandlung in einem Reaktionsgefäß unter Rühren. Vorzugsweise jedoch gibt man die Hydroxylaminlösung über ein Bett des Kationenaustauschers, beispielsweise eine mit dem Kationaustauscher beschickte Säule.

An dem Kationenaustauscher findet in geringem Umfang eine Zersetzung von Hydroxylamin u. a. in N₂ und NH₃ statt. Die entstehenden Gasblasen können die Gleichmäßigkeit des Flüssigkeitsstroms beeinträchtigen und zu einer unerwünschten axialen Rückvermischung führen. Es ist daher besonders bevorzugt, die Hydroxylaminlösung entgegen der Schwerkraftrichtung über ein Bett aus dem Kationenaustauscher zu leiten. So kann man zweckmäßigerweise die Behandlung der Hydroxylaminlösung in einer von unten angeströmten, mit dem Kationenaustauscher beschickten Säule durchführen. Vorzugsweise erfolgt das Anströmen mit einer hohen Strömungsgeschwindigkeit, vorzugsweise einer Strömungsgeschwindigkeit ≥ 10 m/h, insbesondere ≥ 15 m/h (die Strömungsgeschwindigkeit ist das auf den Querschnitt der leeren Säule bezogene durchgesetzte Volumen an Hydroxylaminlösung pro Stunde). Zweckmäßigerweise befindet sich am oberen Ende der Säule ein Rückhalteorgan, wie z. B. eine perforierte Platte oder ein Gestrick, das einen Austrag des Kationenaustauschers verhindert. Diese Maßnahmen führen dazu, dass entstehende Gasblasen fortlaufend aus der Säule ausgetragen werden und der Ionenaustauscher als zusammenhängende Schüttung vorliegt. Damit wird eine unerwünschte Rückvermischung der zu reinigenden Lösung vermieden.

Die Temperatur, bei welcher die Behandlung erfolgt, ist nicht kritisch. Im Hinblick auf die Zersetzlichkeit des Hydroxylamins wird man jedoch höhere Temperaturen vermeiden. Im allgemeinen arbeitet man bei einer Temperatur im Bereich von 0°C bis etwa 50°C, vorzugsweise 20 - 30°C.

Das Mengenverhältnis von zu reinigender Hydroxylaminlösung und Kationenaustauscher hängt von der Menge an zu entfernenden Kationen ab. Der Fachmann kann die geeignete Menge auf einfache Weise durch Verfolgen des Reinigungseffektes bestimmen.

Das erfindungsgemäße Verfahren kann kontinuierlich oder diskontinuierlich durchgeführt werden. Bevorzugt ist die kontinuierliche Betriebsweise.

Das erfindungsgemäße Verfahren ermöglicht es, Hydroxylaminlösungen, die bis etwa 50 ppm, insbesondere bis zu 30 ppm und im allgemeinen 1 bis 10 ppm Metallionen aufweisen, zu reinigen. Bei den Metallionen handelt es sich im allgemeinen um Alkalimetallionen und insbesondere um Natriumionen.

Die mit dem erfindungsgemäßen Verfahren erhaltenen Hydroxylaminlösungen sind im wesentlichen metallionenfrei, das heißt, sie enthalten weniger als 1 ppm, insbesondere weniger als 0,5 ppm Metallionen. Durch ein- oder mehrfache Wiederholung der Behandlung mit einem Kationenaustauscher läßt sich der Metallionengehalt weiter verringern, beispielsweise auf <0,1 ppm. Sie sind daher zur Anwendung in der Elektronikindustrie geeignet.

Im ersten und zweiten Aspekt der Erfindung ist die Behandlung der Hydroxylaminlösung mit einem sauren Kationenaustauscher mit einer Anionenaustauscher-Behandlung kombiniert. Die erfindungsgemäß zu behandelnden wässrigen Hydroxylaminlösungen enthalten in der Regel einen Stabilisator, der eine Zersetzung des Hydroxylamins verhindert bzw. verzögert. Bei den Stabilisatoren handelt es sich meist um anionische Komplexbildner, die zur Komplexierung und Inaktivierung von Schwermetallionen in der Lage sind, welche in unkomplexierter Form potente Katalysatoren der Hydroxylamin-Zersetzung sind. Bei der Behandlung von stabilisierten Hydroxylaminlösungen mit einem Anionenaustauscher wird der Stabilisator an den Anionenaustauscher gebunden. Es besteht daher die Gefahr, dass die mit einem Anionenaustauscher behandelte Hydroxylaminlösung nicht mehr ausreichend stabilisiert ist. Zwar kann der Hydroxylaminlösung nach der Behandlung mit einem Anionenaustauscher erneut Stabilisator zugesetzt werden, doch enthalten die üblichen Stabilisatoren herstellungsbedingt eine mehr oder weniger große Menge Alkalimetall-ionen, insbesondere Natriumionen, so dass durch den nachträglichen Stabilisatorzusatz der Reinigungserfolg einer vorhergegangenen Kationenaustauscherbehandlung weitgehend zunichte gemacht wird.

Zur Entfernung von unerwünschten Anionen in einer zu behandelnden Hydroxylaminlösung geht man gemäß dem ersten Aspekt der Erfindung so vor, dass man die Hydroxylaminlösung mindestens einer Behandlung mit einem Anionenaustauscher in Hydroxyl-Form unterwirft, die behandelte Hydroxylaminlösung mit einem Stabilisator, vorzugsweise einem anionischen Komplexbildner, versetzt und die Hydroxylaminlösung anschließend mindestens einer Behandlung mit einem sauren Kationenaustauscher unterwirft. Alternativ geht man nach dem zweiten Aspekt der Erfindung so vor, dass man die Hydroxylaminlösung mindestens einer Behandlung mit einem Anionenaustauscher in Hydroxyl-Form unterwirft, die Hydroxylaminlösung anschließend mindestens einer Behandlung mit einem sauren Kationenaustauscher unterwirft und die Hydroxylaminlösung mit einer wässrigen Lösung eines Stabilisators, insbesondere eines anionischen Komplexbildners, versetzt, die zuvor zur Entfernung von Metallionen, insbesondere Alkalimetallionen, einer Behandlung mit einem sauren Kationenaustauscher unterworfen wurde.

Brauchbare basische Anionenaustauscher sind beispielsweise die Harze Amberlite IRA-400, IRA-402, IRA-904, IRA-92, IRA-93 und Duolite A-109 der Fa. Rohm & Haas, Dowex 66 und Dowex II der Dow Chemical sowie Purolite A-600, A-400, A-300, A-850 und A-87 und die Lewatit-Typen der Bayer AG. Die Basenforme des Anionenaustauschers kann mit üblichen Basen, wie Natriumhydroxid oder Kaliumhydroxid, erzeugt werden. Die Behandlung mit dem Anionenaustauscher erfolgt in analoger Weise zu der Behandlung mit dem Kationenaustauscher.

Die nachfolgenden Beispiele erläutern die Erfindung, ohne sie zu begrenzen.

### Beispiel 1 (lediglich zur Illustration; nicht erfindungsgemäß)

Die Na-Form des schwachsauren, makroporösen Iminodiessigsäuregruppen-haltigen Ionenaustauschers Lewatit TP 207 (Fa. Bayer), das eine sehr geringe Selektivität bezüglich Na aufweist, wurde durch Spülen mit 0,5 molarer Schwefelsäure mit 5 (ml/h) / ml Ionenaustauscherharz Na-frei gespült. Anschließend wurde so lange mit entionisiertem Wasser schwefelsäurefrei gespült, bis ein pH von 6,5 erreicht wurde.

Dadurch wurden 140 g/h 50 gew.-%ige Hydroxylaminlösung mit 10 ppm Na-Ionen bei Raumtemperatur über eine Ionenaustauscherschüttung von 19,5 ml geleitet. Das Verhältnis Hydroxylaminlösungszufuhr zu Ionenaustauschervolumen betrug 7 (ml/h) / ml. Nach festen Zeiten wurden Fraktionen der gereinigten Lösungen aufgefangen und der Rest-Na-Gehalt bestimmt. Die erhaltenen Fraktionen an gereinigter Lösung enthielten folgende Na-Gehalte:

| | Menge in g | Na-Gehalt in mg/l |
|---|---|---|
| Fraktion 1 | 29,3 | nicht bestimmt |
| Fraktion 2 | 29,6 | 2 |
| Fraktion 3 | 32,7 | 0,2 |
| Fraktion 4 | 33,9 | 0,2 |

Damit wurden 3,4 g HA-Lösung / ml Ionenaustauscher gereinigt.

### Beispiel 2 (lediglich zur Illustration; nicht erfindungsgemäß)

Die nach Beispiel 1 erhaltene Hydroxylaminlösung hatte einen Sulfatgehalt von 30 ppm (30 mg/l). Zur Verringerung des Sulfatgehalts wurde die Lösung über eine Anionenaustauschersäule mit ca. 40 ml Anionenaustauscher in der Hydroxyl-Form geleitet (7 ml/h Hydroxylaminlösung pro ml Anionenaustauscher).

Folgende Ionenaustauscher wurden verwendet:
Amberlite IRA-92
Lewatit M 511
Amberlite IRA-900

In allen Fällen wurde der Sulfatgehalt auf 10 ppm (Nachweisgrenze) reduziert.

## Patentansprüche

1. Verfahren zur Herstellung einer wäßrigen Hydroxylaminlösung, die im wesentlichen metallionenfrei ist, **dadurch gekennzeichnet, daß** man die Hydroxylaminlösung mindestens einer Behandlung mit einem Anionenaustauscher in Hydroxyl-Form unterwirft, die behandelte Hydroxylaminlösung mit einem Stabilisator versetzt und die Hydroxylaminlösung anschließend mindestens einer Behandlung mit einem sauren Kationenaustauscher unterwirft.

2. Verfahren zur Herstellung einer wässrigen Hydroxylaminlösung, die im Wesentlichen metallionenfrei ist, **dadurch gekennzeichnet, dass** man die Hydroxylaminlösung mindestens einer Behandlung mit einem Anionenaustauscher in Hydroxyl-Form unterwirft, die Hydroxylaminlösung anschließend mindestens einer Behandlung mit einem sauren Kationenaustauscher unterwirft und die Hydroxylaminlösung mit einer wässrigen Lösung eines Stabilisators versetzt, die zuvor zur Entfernung von Metallionen einer Behandlung mit einem Kationenaustauscher unterworfen wurde.

3. Verfahren nach Anspruch 1 oder 2, **dadurch gekennzeichnet, daß** man einen schwach-sauren Kationenaustauscher verwendet.

4. Verfahren nach einem der vorhergehenden Ansprüche, **dadurch gekennzeichnet, daß** man einen Kationenaustauscher mit chelatbildenden Gruppen verwendet.

5. Verfahren nach einem der vorhergehenden Ansprüche, **dadurch gekennzeichnet, daß** man die Hydroxylaminlösung über ein Bett aus dem Kationenaustauscher leitet.

6. Verfahren nach Anspruch 5, **dadurch gekennzeichnet, dass** man die Hydroxylaminlösung entgegen der Schwerkraftrichtung über das Bett aus dem Kationenaustauscher leitet.

## Claims

1. A process for the preparation of an aqueous hydroxylamine solution which is essentially free of metal ions, wherein the hydroxylamine solution is subjected to at least one treatment with an anion exchanger in hydroxyl form, a stabilizer is added to the treated hydroxylamine solution, and the hydroxylamine solution is then subjected to at least one treatment with an acidic cation exchanger.

2. A process for the preparation of an aqueous hydroxylamine solution which is essentially free of metal ions, wherein the hydroxylamine solution is subjected to at least one treatment with an anion exchanger in hydroxyl form, the hydroxylamine solution is then subjected to at least one treatment with an acidic cation exchanger, and an aqueous solution of a stabilizer, which was subjected beforehand to a treatment with a cation exchanger to remove metal ions, is added to the hydroxylamine solution.

3. A process as claimed in claim 1 or 2, wherein a weakly acidic cation exchanger is used.

4. A process as claimed in any of the preceding claims, wherein a cation exchanger having chelate-forming groups is used.

5. A process as claimed in any of the preceding claims, wherein the hydroxylamine solution is passed over a bed of the cation exchanger.

6. A process as claimed in claim 5, wherein the hydroxylamine solution is passed opposite to the direction of gravitational force over the bed of the cation exchanger.

## Revendications

1. Procédé pour produire une solution aqueuse d'hydroxylamine essentiellement exempte d'ions métalliques, **caractérisé en ce que** l'on soumet la solution d'hydroxylamine à au moins un traitement avec un échangeur d'anions sous forme hydroxyle, **en ce que** l'on ajoute un stabilisateur à la solution d'hydroxylamine traitée et que l'on soumet ensuite la solution d'hydroxylamine à au moins un traitement avec un échangeur de cations acide.

2. Procédé pour produire une solution aqueuse d'hydroxylamine essentiellement exempte d'ions métalliques, **caractérisé en ce que** l'on soumet la solution d'hydroxylamine à au moins un traitement avec un échangeur d'anions sous forme hydroxyle, **en ce que** l'on soumet ensuite la solution d'hydroxylamine à au moins un traitement avec un échangeur de cations acide, et **en ce que** l'on ajoute à la solution d'hydroxylamine une solution aqueuse d'un stabilisant, laquelle a été soumise préalablement à un traitement avec un échangeur de cations pour éliminer les ions métalliques.

3. Procédé selon la revendication 1 ou 2, **caractérisé en ce que** l'on utilise un échangeur de cations faiblement acide.

4. Procédé selon l'une des revendications précédentes, **caractérisé en ce que** l'on utilise un échangeur de cations avec des groupes chélatants.

5. Procédé selon l'une des revendications précédentes, **caractérisé en ce que** l'on fait passer la solution d'hydroxylamine sur un lit de l'échangeur de cations.

6. Procédé selon la revendication 5, **caractérisé en ce que** l'on fait passer la solution d'hydroxylamine sur le lit de l'échangeur de cations dans le sens contraire à la gravité.
